# EUROPEAN PATENT SPECIFICATION

(11) **EP 2 569 050 B1**
(45) Date of publication and mention of the grant of the patent: **09.08.2017**
(21) Application number: 11721208.4
(22) Date of filing: 13.05.2011
(51) Int. Cl.: A61N 1/36, A61N 1/05, A61F 2/00

(54) **IMPLANTABLE MECHANICAL SUPPORT**
IMPLANTIERBARE MECHANISCHE STÜTZE
SUPPORT MÉCANIQUE IMPLANTABLE

(30) Priority: 13.05.2010 US 334491 P
(43) Date of publication of application: 20.03.2013
(73) Proprietor: AMS Research Corporation, Minnetonka, MN 55343 (US)
(72) Inventor: WATSCHKE, Brian, P., Minnetonka, MN 55343 (US); WANG, Guangjian, Minnetonka, MN 55343 (US)
(74) Representative: Vossius & Partner Patentanwälte Rechtsanwälte mbB
(86) International application number: PCT/US2011/036455
(87) International publication number: WO 2011/143572

(56) References cited:
- WO-A1-01/56499
- WO-A1-01/72240
- WO-A1-2008/130530
- WO-A2-2007/056384
- WO-A2-2007/106303
- WO-A2-2008/111078
- WO-A2-2008/150941
- US-A1- 2006 205 995
- US-A1- 2008 269 548
- US-A1- 2010 023 088

## Description

### FIELD

Embodiments of the invention are directed to a mechanical support of a pelvic treatment apparatus that is configured for implantation in the pelvic region of a patient for the purpose of treating a pelvic condition of the patient, such as urinary incontinence, fecal incontinence, sexual dysfunction, or other pelvic related condition. Additional examples are directed to methods of attaching an electrode lead or an electrical lead to the mechanical support subsequent to the implantation of the mechanical support.

### BACKGROUND

Urinary stress incontinence is a medical condition in which sudden stress placed on a patient's bladder causes urinary incontinence. Activities such as laughing, sneezing, and lifting heavy objects frequently cause stress incontinence in susceptible patients. According to the National Institutes of Health, about 50% of all women have occasional urinary incontinence, and as many as 10% have frequent incontinence. Nearly 20% of women over age 75 experience daily urinary incontinence. Stress incontinence may occur as a result of weakened pelvic muscles that support the bladder and urethra, or because of malfunction of the urethral sphincter. Stress incontinence is often seen in women who have had multiple pregnancies and vaginal childbirths, or who have pelvic prolapse, with cystocele, cystourethrocele, or rectocele (protrusion of the bladder, urethra, or rectal wall into the vaginal space). Risk factors for stress incontinence include female sex, advancing age, childbirth, smoking, and obesity.

Urge incontinence involves a strong, sudden need to urinate, immediately followed by a bladder contraction, resulting in an involuntary loss of urine. The bladder's ability to fill and store urine requires a functional sphincter (muscle controlling output) and a stable bladder wall muscle (detrusor). Undesired bladder muscle contraction may occur as the result of a break in a neurological pathway from the brain to the bladder. It can also occur if the bladder is irritated and the normal neurological impulses to inhibit urination are insufficient to keep the bladder relaxed as it fills. Urge incontinence may result from neurological injuries (such as spinal cord injury or stroke), neurological diseases (such as multiple sclerosis), infection, bladder cancer, bladder stones, bladder inflammation, or bladder outlet obstruction.

Although urge incontinence may occur in anyone at any age, it is more common in women and the elderly. It is second only to stress incontinence as the most common cause of urinary incontinence (involuntary loss of urine), and it is common for people with stress incontinence to also have urge incontinence.

In the urology field, needles, suture passers and ligature carriers are utilized in a variety of procedures, many of which are designed to treat incontinence. A pubovaginal sling procedure is a surgical method involving the placement of a sling to stabilize or support the bladder neck or urethra. There are a variety of different sling procedures. Descriptions of different sling procedures are disclosed in U.S. Pat. No. 5,112,344 to Petros et al., U.S. Pat. No. 5,611,515 to Benderev et al., U.S. Pat. No. 5,842,478 to Benderev et al., U.S. Pat. No. 5,860,425 to Benderev et al., U.S. Pat. No. 5,899,909 to Claren et al., U.S. Pat. No. 6,039,686 to Kovac, U.S. Pat. No. 6,042,534 to Gellman et al., and U.S. Pat. No. 6,110,101 to Tihon et al.

Some pubovaginal sling procedures extend a sling from the rectus fascia in the abdominal region, to a position below the urethra, and back again to the rectus fascia. The Tension-free Vaginal Tape (TVT) procedure (Ethicon, N.J.) utilizes a Prolene^{™} nonabsorbable, polypropylene mesh. Additional sling procedures are disclosed in US Patent Application Publication 2001/0018549A1 to Scetbon, and PCT Patent Publications WO 02/39890 to Ulmsten et al. and WO 02/069781 to Ulmsten et al. . Other examples of a mesh sling used for treating incontinence include the In-Fast^{™} and InVance^{™} mesh sling (American Medical Systems, Inc., Minnetonka, Minn.), which are the subject of various patents and applications. In general, these systems use bone anchors to affix the mesh sling to the pubic bone in a manner that compresses the urethra.

The BioArc^{™} SP Sling System and the BioArc TO Subfascial Hammock (American Medical Systems) treat female stress incontinence with a minimally invasive procedure using either a suprapubic (BioArc SP) or a transobturator (BioArc TO) approach. The BioArc utilizes a polypropylene mesh for fixation and a biologic graft material for suburethral support. Tensioning and loosening sutures maintain mesh integrity during placement and allow for intra-operative tensioning refinement without distorting the biologic graft material.

Both the BioArc SP and BioArc TO have two pieces of precut polypropylene mesh with attached clamps. The mesh and clamps are designed to facilitate the attachment of the surgeon's choice of graft materials. Tensioning and loosening sutures allow for intra-operative tension adjustment without mesh or biologic distortion. The BioArc is minimally invasive, and patients typically experience immediate continence following surgery.

The Monarc^{™} Subfascial Hammock^{™} (American Medical Systems) is a minimally invasive treatment option for female patients suffering from stress incontinence. The Monarc treats stress incontinence by supporting the urethra during abdominal stress events, such as sneezing, coughing or laughing. Monarc's outside-to-in trans-obturator approach avoids the retropubic space. A knitted, polypropylene mesh creates a hammock-shaped midurethral sling, and helps to maintain continence by supporting the midurethra during times of increased abdominal pressure. Knitted, polypropylene Type I mesh offers tissue fixation without suturing and large pores to facilitate tissue integration. The tensioning suture maintains mesh integrity during placement and allows for intra- and immediate post-operative tensioning, while reducing sling deformation. After the procedure, most patients experience immediate continence.

The SPARC^{™} Self-Fixating Sling System (American Medical Systems) utilizes a suprapubic approach to treat female stress incontinence due to urethral hypermobility and/or intrinsic sphincter deficiency (ISD). It utilizes a self-fixating sling with a resorbable tensioning suture that allows for intra-operative tension refinement without mesh distortion, as well as immediate post-operative adjustment prior to tissue ingrowth. The sling supports the urethra during increased abdominal pressure (e.g., sneezing, coughing or laughing). Large pores of the mesh promote tissue integration for healing. The mesh sling is covered with a plastic sheath, and has locking connectors attached. SPARC's resorbable tensioning suture maintains mesh integrity during placement and allows for intra- and immediate post-operative tensioning, while reducing sling deformation. Patients typically experience immediate continence following the implantation procedure.

The In-Fast^{™} Ultra Transvaginal Sling (American Medical Systems) is a minimally invasive treatment for female stress incontinence. To place a sling, the In-Fast Ultra inserter is used to drive two small titanium bone screws into the posterior aspect of the pubic bone. Each screw has a suture attached that facilitates attachment to a biologic graft or synthetic mesh that is placed under the urethra, thus restoring lost pubocervical support. The graft or mesh provides support for the urethra to restore continence. The In-Fast produces immediate continence in most cases.

U.S. Pat. Nos. 6,354,991 and 6,896,651 to Gross et al., describe a device and method for treatment of urinary stress incontinence. At least one electrode is implanted in a pelvic muscle of a patient. A control unit receives signals indicative of abdominal stress in the patient and responsive thereto applies an electrical waveform to the electrode which stimulates the muscle to contract, so as to inhibit involuntary urine flow through the patient's urethra due to the stress.

U.S. Pat. No. 6,652,449 to Gross et al., describes a device for treatment of a patient's urinary incontinence, including a sensor, which generates a signal responsive to a physiological characteristic indicative of a likelihood of incontinence. A control unit receives the signal from the sensor. At least one electrode is preferably implanted in the patient. The electrode is coupled to cause contraction of the pelvic muscle of the patient responsive to application of electrical energy to the electrode. Responsive to the signal, the control unit applies an electrical waveform to the electrode, so as to inhibit the incontinence.

U.S. Pat. No. 6,862,480 to Cohen et al., describes a device for treating a medical condition. A surgical procedure for implanting the device is also disclosed. The device includes a sensor, which is adapted to generate a signal responsive to a state of a patient, and at least one electrode, which is adapted to be coupled to a pelvic site of the patient. A control unit is adapted to receive the signal, to analyze the signal so as to distinguish between an imminent stress incontinence event and an imminent urge event, and, responsive to analyzing the signal, to apply an electrical waveform to the at least one electrode. In various configurations, the device may be used alternatively or additionally to treat fecal incontinence, interstitial cystitis, chronic pelvic pain, or urine retention.

U.S. Pat. No. 6,941,171 to Mann et al., describes a method and system for treatment of incontinence, urgency, frequency, and/or pelvic pain. The method includes implantation of electrodes on a lead or the discharge portion of a catheter adjacent the perineal nerve(s) or tissue(s) to be stimulated. Stimulation pulses, either electrical or drug infusion pulses, are supplied by a stimulator implanted remotely, and through the lead or catheter, which is tunneled subcutaneously between the stimulator and stimulation site. For instance, the system and method may reduce or eliminate the incidence of unintentional episodes of bladder emptying by stimulating nerve pathways that diminish involuntary bladder contractions, improve closure of the bladder outlet, and/or improve the long-term health of the urinary system by increasing bladder capacity and period between emptying. Moreover, the system and method may allow a patient to be taught to receive one or more patterns of neural stimulation that can be prescribed by a physician and administered without continuous oversight by a clinical practitioner.

U.S. Pat. No. 6,135,945 to Sultan, describes apparatus for preventing uncontrolled discharge of urinary fluid from a patient urethra. The apparatus includes a pressure sensor operable for sensing intra-abdominal pressure when implanted in a patient and operable for generating a pressure signal in response to the sensed pressure. An actuating device is operably coupled to the pressure sensor and is responsive for generating an electrical signal in response to the pressure signal. A controller is operably coupled to the actuating device and configured for engaging the urethra to selectively compress the urethra and prevent incontinence. The controller includes a plate adapted to be positioned on one side of the urethra and an element adapted to be positioned on another side of the urethra, and is operable for varying the distance between the element and the plate in response to the electrical signal to compress the urethra therebetween so that incontinence is prevented when intra-abdominal pressure is experienced by a patient. In an embodiment, the controller is simply an electrode placed inside the wall of the urethral sphincter to enhance its contraction and tone during the increase in the intra-abdominal pressure.

In an embodiment described in the 945 patent, apparatus for preventing uncontrolled discharge of urinary fluid from a patient urethra includes (a) a pressure sensor operable for sensing intra-abdominal pressure when implanted in a patient and operable for generating a pressure signal in response to the sensed pressure; (b) an actuating device operably coupled to the pressure sensor, the actuating device responsive for generating an electrical signal in response to the pressure signal; and (c) a controller operably coupled to the actuating device and configured for engaging the urethra to selectively compress the urethra and prevent incontinence. The controller comprises a sling adapted to be positioned on one side of the urethra, a movable metal element coupled to the sling, and an electromagnetic element positioned proximate the metal element, the electromagnetic element creating a magnetic field proximate the metal element in response to the electrical signal for magnetically moving the metal element and thereby directing the sling against the urethra to compress the urethra so that incontinence is prevented when intra-abdominal pressure is experienced by a patient.

U.S. Pat. No. 5,112,344 to Petros et al., describes a method of treating female incontinence comprising looping a filamentary element between the wall of the vagina and the rectus abdominis sheath in the anterior wall of the abdomen whereby it passes to each side of the urethra into the correct spatial relationship to the pubis, allowing the development of scar tissue between the vaginal wall and the rectus abdominis sheath, and removing the filamentary element. A surgical instrument for use with the method comprises a surgical instrument for the application of a filamentary element into the body for the purpose of treating female incontinence, the instrument comprising a tubular shaft having a handle at one end and carried toward its other end a flexible needle element slidably receivable in the shaft and adapted at one end to receive a filamentary element and having an enlarged profiled portion at its other end whereby when the needle element is received in the shaft the other end of the needle element defines a convergent surface of the other end of the shaft and the one end of the needle element is exposed at the one end of the shaft.

U.S. Pat. Nos. 5,611,515, 5,842,478 and 5,860,425 to Benderev et al., describe a surgical treatment of stress urinary incontinence. The described methods include: 1) a technique of probe passage intended to avoid injuring the bladder and to provide a more accurate and reproducible capture of the pubocervical fascia lateral to the bladder neck and urethra, 2) anchor fixation of the suspending sutures to the pubic bone intended to decrease the risk of suture pull through from above and to decrease post-operative pain and 3) a technique intended to simply and reproducibly set a limited tension of the suspending sutures. A description of these methods and results of procedures with some of these methods are disclosed. Drill guides, suture passers, suture tensioners, and various related tools and devices for use in the surgical method are also described.

U.S. Pat. No. 6,039,686 to Koval, describes a pubic bone-mounted urethra stabilization and support system and method therefor for the long term cure of recurrent female urinary incontinence. The system comprises a pair of anchors affixed to the posterior/inferior pubic bone, sutures attached to the anchors, and a mesh sling passing behind and about the urethra and the adjacent endopelvic fascia and having ends attached to the anchors by the anchor-mounted sutures. The method includes the steps of accessing the urethra with the endopelvic fascia therebehind and the pubic bone, properly locating and attaching the anchors to the pubic bone, properly locating the sling about the urethra and adjacent endopubic fascia and suturing and tensioning the ends of the sling to the anchors, intending to cause the sling to restore, support and stabilize functional urethral continence anatomy and intending to prevent urethral descent under intraabdominal pressure.

U.S. Pat. No. 6,042,534 to Gellman et al., describes prefabricated urethra suspension slings, methods of making the slings, methods of attaching suture to the slings, kits comprising the slings, and methods of using the slings to treat urinary incontinence. The slings comprise a biocompatible material having an elongate shape adapted for urethral suspension. The material has a central portion extending longitudinally between a first end portion and a second end portion. Each end portion of the sling contains at least one suture receiving site. The suture receiving sites are formed prior to surgery and may be reinforced through a variety of means. Sutures may be attached to the suture receiving sites during the manufacturing process or by the physician prior to or during surgery. Additionally, the end portions of the sling containing the suture receiving sites may be thicker than the central portion of the sling.

U.S. Pat. No. 6,110,101 to Tihon et al., describes a sling for supporting the urethra and neck of the bladder to prevent urinary incontinence. The sling is intended to provide sufficient support to inhibit the unintended flow of urine, yet stretch in a controlled fashion so that the bladder can be voided at appropriate times.

US Patent Application 2001/0018549 to Scetbon, describes a device for treating urinary stress incontinence in women, including a flexible and elongate mechanism including a tape for supporting a urethra and a flat protective sheath enveloping the tape; and a puncturing needle with an active distal end and a proximal end connected to a first end of the flexible and elongate mechanism, wherein the proximal end of the puncturing needle is connected to a first end of the flexible and elongate mechanism by an intermediate traction element, a second end of the flexible and elongate mechanism being free. A method for treating urinary stress incontinence in a woman suffering from urinary stress incontinence is also described, including (a) forming an opening in an anterior vaginal wall; (b) creating, from two small suprapubic incisions formed in the abdominal wall, a right track and a left track from the abdominal skin to the opening formed in the anterior vaginal wall; (c) using a needle and an intermediate traction element to follow one of the tracks and following the other track with at least a needle; (d) verifying by cystoscopy that the paths of the tracks are outside the bladder and the urethra; (e) using a support tape surrounded by a plastic sheath to follow the tracks by passage under an inferior surface of the urethra; (f) adjusting a loop formed by the sheathed tape under the inferior surface of the urethra; (g) removing the sheath by pulling the sheath toward the outside of the woman's body through the small suprapubic incisions; and (h) leaving the tape implanted from the first to the second incision and around the urethra to support the urethra.

U.S. Pat. No. 5,899,909 to Claren et al., describes a surgical instrument and a method for treating female urinary incontinence. The instrument comprises a shank having a handle at one end thereof, and two curved needle-like elements which are connected at one end thereof, each with one end of a tape intended to be implanted into the body. These elements can be connected one at a time with the shank at the other end thereof to form a curved end portion of the shank and are intended to be passed into the body via the vagina, each element being dimensioned to extend from the inside of the vaginal wall over the back of the pubic bone to the outside of the abdominal wall. When practicing the method, the tape is passed into the body via the vagina first at one end and then at the other end, at one side and the other, respectively, of the urethra to form a loop around the urethra, located between the urethra and the vaginal wall. The tape is extended over the pubis and through the abdominal wall and is tightened. Then, the tape ends are cut at the abdominal wall, and the tape is left implanted in the body.

PCT Patent Publication WO 02/39890 to Ulmsten et al., describes a surgical instrument and a method for treating female urinary incontinence. The instrument comprises a handle mechanism and one or two curved needle-like elements which are connected at opposite ends of a length of tape, which is implanted into the body. These elements can be connected one at a time with the handle and are intended to pass into the body via the vagina, each needle-like element being dimensioned to extend from the inside of the vaginal wall, under the pubic bone and to the outside of the abdominal wall. When practicing the method, the tape is passed into the body via the vagina first at one end and then at the other end, at one side and the other, respectively, of the urethra to form a loop around the urethra, located between the urethra and the vaginal wall. The tape is extended under the pubis and through the abdominal wall and adjusted. The tape ends are cut at the abdominal wall, and the tape is left implanted in the body.

PCT Patent Publication WO 02/069781 to Ulmsten et al., describes a surgical instrument and a method for treating female urinary incontinence. A tape or mesh is permanently implanted into the body as a support for the urethra. In one embodiment, portions of the tape comprise tissue growth factors and adhesive bonding means for attaching portions of the tape to the pubic bone. In a further embodiment, portions of the tape comprise attachment means for fastening portions of the tape to fascia within the pelvic cavity. In both embodiments, the tape is implanted with a single incision through the vaginal wall.

U.S. Pat. No. 6,652,450 to Neisz et al., describes an implantable article and method of use to treat urological disorders. A biocompatible device includes a sling assembly intended to be minimally invasive and provide sufficient support to the target site. In addition, the configuration of the sling assembly is intended to allow the position of the sling to be permanently changed during and/or after implantation.

U.S. Pat. No. 6,612,977 to Staskin et al., describes an apparatus and method of use to treat urological disorders. A biocompatible device includes a handle, needle, dilator and sling assembly intended to be minimally invasive and provide sufficient support to the target site. In addition, the configuration of the sling assembly is intended to allow the sling to be adjusted during and/or after implantation.

U.S. Pat. No. 6,802,807 to Anderson et al., describes an apparatus and method of use to treat urological disorders. The device is a repositionable handle for an arcuate needle intended to be minimally invasive. The device is for use with a sling assembly that allows a sling to be controllably implanted in a position.

U.S. Pat. No. 6,911,003, US Patent Application Publication 2003/0171644 and US Patent Application Publication 2005/0143618 to Anderson et al., describe surgical articles, implants and components suitable for a transobturator surgical procedure.

US Patent Application Publication 2005/0245787 to Cox et al., describes a method of treating pelvic organ prolapse. The method generally includes the steps of establishing a first pathway between the external perirectal region of the patient to the region of the ischial spine in tissue on one side of the prolapsed organ, followed by establishing a second pathway in tissue on the contralateral side of the prolapsed organ. A support member, which includes a central support portion and two end portions, is described as being positioned in a position to reposition the prolapsed organ in the organ's anatomically correct location. The end portions of the support member are described as being introduced through the respective tissue pathways, followed by adjustment of the end portions so that the support member is located in a therapeutic relationship to the prolapsed organ that is to be supported. An apparatus and kit for the treatment is further described.

US Patent Application 2005/0250977 to Montpetit et al., describes a method for cystocele repair comprising the steps of: establishing four pathways in tissue around a bladder of a patient, introducing a strap into each of the pathways, and positioning beneath the bladder of the patient a support member having each strap connected thereto such that the bladder of the patient is supported by the support member and a bulge of the bladder into a vagina of the patient is reduced.

U.S. Pub. No. 2007/0260288 A1 to Gross, describes a pelvic treatment apparatus that comprises an implantable mechanical support, such as a sling, that is shaped to support a portion of a urethra of a patient or another pelvic structure. One or more electrodes are coupled to the mechanical support, so as to contact tissue of the patient. A control unit drives the electrodes to apply a current to the tissue. The mechanical support is typically configured to treat stress incontinence or another condition in which it is desired to support the urethra, and the applied current is typically configured to treat urge incontinence.

### SUMMARY

Embodiments of the invention are generally directed to a mechanical support of a pelvic treatment apparatus that is configured for implantation in the pelvic region of a patient for the purpose of treating a pelvic condition of the patient, such as urinary incontinence, fecal incontinence, sexual dysfunction, or other pelvic related condition. The present invention is defined in the claims. According to the invention, the implantable mechanical support comprises a section of mesh having a longitudinal axis and at least one radiopaque mark covering a subsection of the section of mesh.

In some embodiments, the mark comprises a line extending approximately perpendicularly to the longitudinal axis. In some embodiments, the mark comprises at least 3 separate marks, and each of the marks is evenly spaced from adjacent marks along the longitudinal axis. In some embodiments, the radiopaque mark has a shape that can provide feedback to the physician as to the orientation of the structure to which the radiopaque mark is attached relative to a radiological image of the implanted mechanical support.

In some embodiments, the mechanical support comprises a lead connector that is attached to the mesh adjacent the mark. The lead connector is configured to couple an electrode lead to the section of mesh. In some embodiments, the lead connector comprises an opening that is configured to receive an electrode lead, and the mark indicates the location of the opening. In some embodiments, the lead connector is in the form of a clip, a tube or a threaded member.

In some embodiments of the mechanical support, one or more electrodes, one or more electrical conductors, and an electrical connector are attached to the mesh. The electrical connector is electrically coupled to the one or more electrodes through the one or more conductors. In one embodiment, the mark indicates the location of the one or more electrodes, the one or more electrical conductors, and/or the electrical connector.

In some embodiments, the mechanical support comprises a plurality of electrodes attached to the section of mesh. A plurality of electrical connectors are each electrically coupled to a different subset of the plurality of electrodes. In one embodiment, the mark comprises a plurality of marks each indicating the location of one of the electrical connectors. In some embodiments, each of the plurality of marks indicates a location of at least one of the electrical connectors, the subset of the electrodes electrically coupled to the electrical connector, and/or the electrical conductors electrically coupled to the electrical connector.

In some embodiments, the section of mesh of the mechanical support comprises first and second portions adjacent first and second ends, and an expandable portion extending between the first and second portions. The expandable portion comprises a compact state, in which the expandable portion comprises a plurality of folds, and an expanded state, in which a length of the expandable portion along the longitudinal axis is greater than the length of the expandable portion when in the compact state. In one embodiment, the expandable portion does not undergo plastic deformation when in the expanded state, or when transitioned from the compact state to the expanded state.

Some embodiments are directed to a method, in which an implantable mechanical support is provided. The mechanical support comprises a section of mesh having a longitudinal axis and a radiopaque mark covering a subsection of the section of mesh. Also in the method, the mechanical support is implanted adjacent a target site of a patient. In accordance with some embodiments, the implantation of the mechanical support comprises aligning the mark relative to the target site using a radiological image. The radiological image may be obtained using a fluoroscope, x-ray, or through another suitable radiological imaging technique.

In some embodiments of the method, an incision is made in the patient and a distal end of an electrode lead is fed through the incision toward the implanted mechanical support. The radiopaque mark is then located on the mesh using a radiological image. The distal end of the electrode lead is then attached to the mesh proximate to the mark. In some embodiments, this attachment of the lead to the mesh adjacent the mark is facilitated by an anchor of the electrode lead, such as a helical coil or other suitable anchor.

In some embodiments, the implantable mechanical support further comprises a lead connector attached to the mesh adjacent the mark. In the method, a distal end of an electrode lead is fed into the patient and toward the implanted mechanical support. The lead connector is located by locating the mark using a radiological image. The electrode lead is then attached to the implanted mechanical support using the lead connector. In some embodiments, the lead connector comprises an opening and the mark is positioned to indicate the location of the opening of the lead connector. In the method, the opening of the lead connector is located by locating the mark using a radiological image. The electrode lead is then attached to the implanted mechanical support by inserting the electrode lead through the opening of the connector.

In some embodiments, the implantable mechanical support comprises one or more electrodes attached to the mesh and an electrical connector attached to the mesh adjacent the mark and electrically coupled to the one or more electrodes. In the method, a distal end of an electrical lead is fed through the patient and toward the implanted mechanical support. The electrical connector is located by locating the mark using a radiological image. The distal end of the electrical lead is then attached to the electrical connector to form an electrical connection between the electrical lead and the one or more electrodes.

In some embodiments of the above-described methods, the feeding of the electrode lead or electrical lead, the locating of the radiopaque mark, the lead connector or the electrical connector, and the attachment of the electrode lead to the mesh, the lead connector or the electrical connector, occurs more than 1 week after implanting the mechanical support.

In some embodiments, the target site of the method is adjacent the urethral sphincter, the anal sphincter or a muscle of the pelvic floor.

Some embodiments are directed to a method of attaching a lead to (e.g., an electrode lead or an electrical lead) to a mechanical support, which comprises mesh implanted in a patient. In the method, an incision is made in a patient and the lead is fed through the incision toward the implanted mesh. A radiopaque mark on the mesh is located using a radiological image. A distal end of the lead is then attached to the implanted mesh adjacent the mark. In some embodiments, the distal end of the lead is attached to the mesh by attaching the lead to a lead connector, which is attached to the mesh adjacent the mark. In accordance with other embodiments, the distal end of the lead is attached to the mesh by attaching the lead to an electrical connector, which is attached to the mesh and is electrically coupled to one or more electrodes attached to the mesh.

Some embodiments are directed to an implantable mechanical support that comprises first and second mesh sections, and an expandable section extending between the first and second mesh sections. In some embodiments, the expandable section comprises a compact state in which the expandable section comprises a plurality of folds. The expandable section also includes an expanded state, in which a length of the expandable section along a longitudinal axis is greater than the length of the expandable section when it is in the compact state. In some embodiments, the expandable section does not undergo plastic deformation when in the expanded state or when transitioned from the compact state to the expanded state. In some embodiments, one or more electrodes are attached to at least one of the first mesh section, the second mesh section, or the expandable section. In some embodiments, the expandable section comprises at least one electrical conductor coupled to the one or more electrodes. In some embodiments, the mechanical support comprises an absorbable suture that is connected to the first and second mesh sections. The absorbable suture maintains the expandable section in the compact state. When the mechanical support is implanted in a patient, the absorbable suture dissolves away over time releasing the expandable section from its compact state and allowing it to expand within the tissue of the patient.

In some embodiments, the expandable section is biased to maintain the compact state. That is, the expandable section requires a tensile force to move it from a quiescent compact state to the expanded state.

Some embodiments are directed to a method, in which an implantable mechanical support is provided. In one embodiment, the mechanical support comprises first and second mesh sections, and an expandable section extending between the first and second mesh sections. The expandable section comprises a compact state, in which the expandable section comprises a plurality of folds. The expandable section also comprises an expanded state, in which a length of the expandable section along a longitudinal axis is greater than the length of the expandable section when in the compact state. Also in the method, the mechanical support is implanted within a patient proximate to a target site. The first mesh section, the second mesh section, and the expandable section are then placed in tension along the longitudinal axis and the length of the expandable section is responsively expanded. The ends of the first and second sections are then secured to the patient.

Additional embodiments are directed to an implantable mechanical support that comprises a section of mesh and a lead connector attached to the mesh. The lead connector is configured to couple a lead (e.g., electrode lead) to the mesh. In one embodiment, the lead connector comprises a radiopaque mark that may be imaged using a radiological technique. In some embodiments, the lead connector comprises an opening configured to receive the lead and the radiopaque mark indicates a location of the opening.

Some embodiments are directed to an implantable mechanical support that comprises a section of mesh, one or more electrodes attached to the section of mesh, one or more electrical conductors attached to the mesh, and an electrical connector attached to the mesh. The electrical connector is electrically coupled to the one or more electrodes through the one or more conductors, wherein the electrical connector comprises a radiopaque mark. In some embodiments, the electrical connector comprises an opening configured to receive an end of an electrical lead, and the radiopaque mark indicates a location of the opening.

### BRIEF DESCRIPTION OF THE DRAWINGS

FIGS. 1 and 2 are schematic illustrations of a pelvic treatment apparatus 100 in accordance with some embodiments of the invention.
FIG. 3 is a simplified diagram of an exemplary stimulation system that may be used to provide a stimulation therapy to a target site of a patient.
FIG. 4 is a schematically illustrated side view of an anchor of an electrode lead attached to an implantable mechanical support, in accordance with embodiments of the invention.
FIG. 5 is a schematically illustrated side view of an electrode lead attached to an implantable mechanical support in accordance with an exemplary embodiment of the invention.
FIG. 6 is a schematically illustrated top view of a lead connector of a mechanical support securing a distal end of an electrode lead to the mechanical support, in accordance with embodiments of the invention.
FIGS. 7 and 8 respectively are schematically illustrated top and side views of a lead connector of a mechanical support in accordance with embodiments of the invention.
FIGS. 9 and 10 respectively are schematically illustrated top and side views of a lead connector of a mechanical support in accordance with embodiments of the invention.
FIG. 11 is a schematically illustrated top view of a lead connector of a mechanical support in accordance with embodiments of the invention.
FIG. 12 is a schematically illustrated top view of a portion of a mechanical support in accordance with embodiments of the invention.
FIG. 13 is a schematically illustrated top view of a portion of a mechanical support in accordance with embodiments of the invention.
FIGS. 14 and 15 are a schematically illustrated top views of a mechanical support in accordance with embodiments of the invention respectively in compact and expanded states, respectively.
FIGS. 16 and 17 are schematically illustrated side views of a portion of the mechanical support of FIGS. 14 and 15, respectively.
FIGS. 18-22 are simplified views of a pelvic region of a female patient illustrating method steps in accordance with embodiments of the invention.

Elements having the same or similar labels in the drawings correspond to the same or similar elements.

### DETAILED DESCRIPTION OF ILLUSTRATIVE EMBODIMENTS

Some embodiments of the present invention are directed to a pelvic treatment apparatus that is configured for implantation in the pelvic region of a patient for the purpose of treating a pelvic condition of the patient, such as urinary incontinence, fecal incontinence, sexual dysfunction, or other pelvic related conditions.

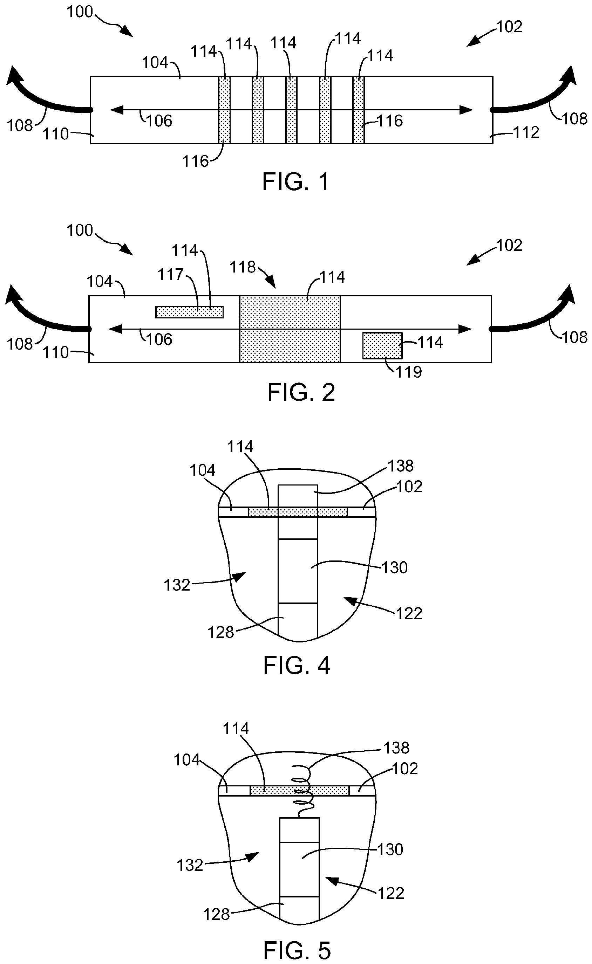

FIGS. 1 and 2 are schematic illustrations of a pelvic treatment apparatus 100 in accordance with some embodiments of the invention. In one embodiment, the pelvic treatment apparatus 100 comprises an implantable mechanical support 102 comprising a section of mesh 104 having a longitudinal axis 106. In one embodiment, the mesh 104 is in the form of a sling that is shaped to support and/or compress a portion of the urethra, or other pelvic structure of the patient. In one embodiment, the mechanical support 102 comprises a pair of anchors 108 attached to opposing ends 110 and 112 of the mesh 104. Embodiments of the anchors 108 include conventional anchors used to attach the mesh 104 to bone (e.g., pubic bone) or tissue of the patient.

In one embodiment, the mechanical support 102 comprises one or more radiopaque marks 114. The marks 114 may be visualized by a physician using a suitable radiological imaging technique, such as fluoroscopy. The marks 114 are visible in radiological images of the support, which may be obtained using a fluoroscope (or x-ray) during implantation of the support 102 in the patient, or subsequent to the implantation of the support 102 in the patient. In one embodiment, the marks 114 are distinguishable from the mesh 104 in the radiological image. As discussed in greater detail below, the marks 114 may be used to assist in the positioning of the mesh 104 in the desired location relative to pelvic structures during implantation, to determine the location of the mesh or features thereof post implantation, to locate particular features of the mesh 104 for the purpose of attaching an electrode lead or an electrical lead to the mesh 104 post implantation of the support 102, and/or other to provide other benefits.

In some embodiments, the radiopaque marks 114 are formed on a surface of the mesh 104 by applying a radiopaque material to the mesh 104 through a printing or other suitable process. In accordance with other embodiments, the radiopaque marks 114 may be formed by interweaving radiopaque material within the fibers of the mesh 104. In one embodiment, the radiopaque material comprises barium sulfate or other suitable radiopaque material.

The marks 114 may take on many different forms. In one embodiment, the marks 114 are configured to assist in aligning the mesh 104 relative to another object, such as an anatomical feature of the patient, such as the urethra. In one embodiment, the one or more marks 114 are in the form of a line 116 that extends transversely to the longitudinal axis 106, as shown in FIG. 1. In one embodiment, the line 116 extends approximately perpendicularly to the longitudinal axis 106. In accordance with another embodiment, the marks 114 comprise a line 117 that is oriented substantially parallel to the longitudinal axis 106, as shown in FIG. 2.

In one embodiment, the radiopaque marks consist of a shape that could provide feedback to the physician as to the orientation of the implant relative to the radiological image. For example, the mark could consist of a circle such that when the radiological image is taken on face to the implant and radiopaque mark, it appears as a circle in the image. However, if it were to be viewed from an oblique angle, the mark would appear as an oval in the radiological image. Being able to discern the orientation of the implant could be used to aide the physician in the docking of the electrode lead or electrical lead with the implant.

In accordance with another embodiment, the one or more marks 114 comprise at least 3 separate marks 114 that are evenly spaced from adjacent marks along the longitudinal axis 106, as shown in FIG. 1, or along a line extending perpendicular to the longitudinal axis 106. This spacing of the marks 114 can assist a physician in aligning the support 102 during implantation and/or locating anatomical features of the patient relative to the mesh 104 post implantation of the support 102.

In one embodiment, one of the marks 114 is located in an intermediary section 118 of the mesh 104. In accordance with another embodiment, one or more of the marks 114, such as mark 119 is located toward one of the ends 110 or 112 of mesh 104.

As mentioned above, some embodiments of the invention assist in the attachment of an electrode lead to the support 102 subsequent to the implantation of the mechanical support 102 in a patient. This may be necessary when, for example, there is a desire to apply electrical stimulation to the patient in the region of the implanted mesh 104 to treat a pelvic condition of the patient, such as urinary incontinence, fecal incontinence sexual dysfunction, or other pelvic related condition. Once the electrode lead is attached to the mesh 104, electrical stimulation therapy for treating the desired pelvic condition of the patient may be applied to the target site adjacent the mesh 104 in accordance with conventional techniques, such as those described above.

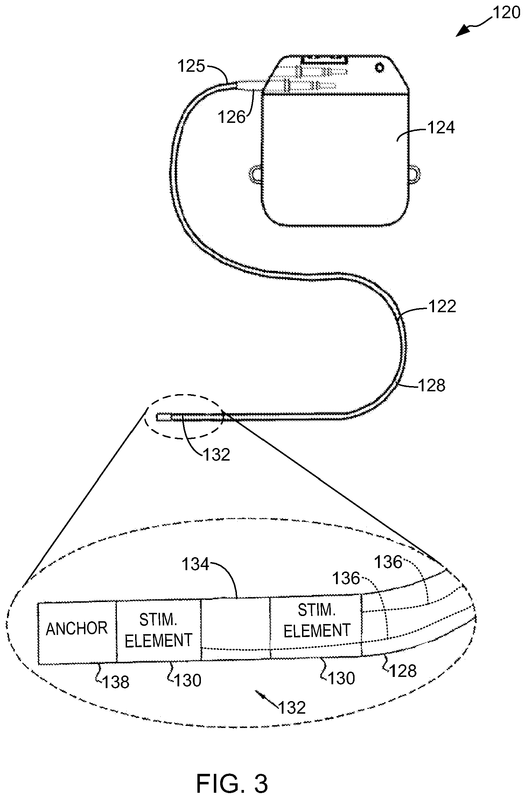

FIG. 3 is a simplified diagram of an exemplary stimulation system 120 that may be used to provide the desired stimulation therapy to a target site adjacent the mesh 104 of the mechanical support 102 after the mechanical support 102 has been implanted in the patient. In one embodiment, the system 120 comprises an electrode lead 122 that is configured to electrically couple to a control unit 124 at a proximal end 125 via a connector 126. The electrode lead 122 comprises a lead body 128 and one or more stimulation elements or electrodes 130 attached to a distal end 132 of the lead body 128. In one embodiment, the electrodes 130 are separated from each other by an insulative portion or element 134. The lead body 128 insulates electrical wires 136 connecting the control unit 124 to the electrodes 130. The lead body 128 can be in the form of an insulating jacket typically comprising silicone, polyurethane or other flexible, biocompatible electrically insulating material.

In one embodiment, the control unit 124 comprises circuitry including at least one processor for processing electrical signals received from the one or more electrodes 130 or physiological sensors (not shown). In one embodiment, the control unit 124 is also configured to apply an electrical current or waveform to tissue of the patient through the one or more electrodes 130 in contact with the tissue. In one embodiment, the control unit 124 receives power from an internal battery (not shown). In one embodiment, the control unit 124 is configured for implantation in the patient. In one embodiment, the control unit 124 represents a stimulating device that is external to the patient, such as one used for preliminary testing of a stimulation therapy on the patient.

In one embodiment, the electrode lead 122 comprises one or more anchors 138 at the distal end 132. One embodiment of the anchor 138 is configured to attach to the mesh 104 of the implantable mechanical support 102, as illustrated schematically in FIG. 4. Embodiments of the anchor 138 include tines or other suitable anchor that can be entangled with the implanted mesh 104. In one exemplary embodiment, the anchor 138 comprises a helical coil, which can pierce or be screwed into the mesh 104 to anchor the distal end 132 of the electrode lead 122 to the implanted mesh 104, as shown in FIG. 5. In one embodiment, the mesh 104 of the implantable support 102 comprises a radiopaque mark 114 that identifies a location on the mesh 104 where the distal end 132 of the electrode lead 122 is to be attached, as shown in FIG. 4.

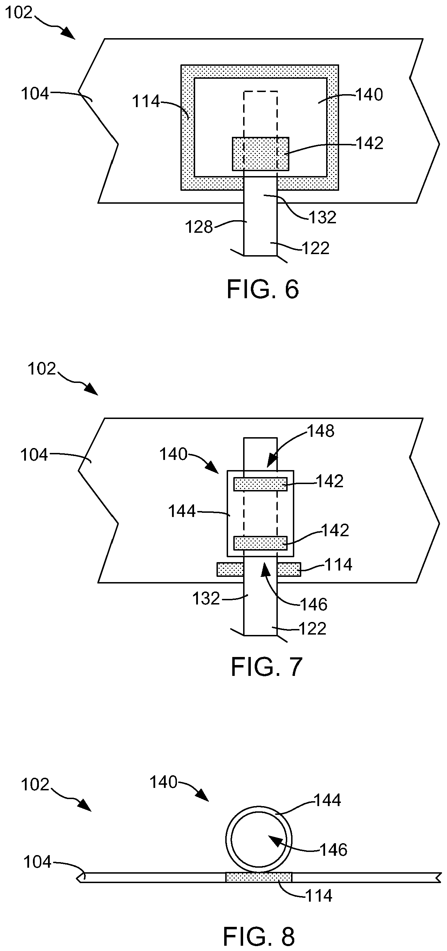

In accordance with some embodiments, the mechanical support 102 comprises a lead connector 140 attached to the mesh 104 as illustrated schematically in FIG. 6. In one embodiment, the lead connector 140 is configured to secure the distal end 132 of the electrode lead 122 to the mesh 104. In one embodiment, the mesh 104 comprises a radiopaque mark 114 that identifies the location of the lead connector 140. For instance, the mark 114 on the mesh 104 may surround the lead connector 140, or be positioned adjacent to the lead connector 140. The radiological image of the mark 114 can assist the physician in locating the connector 140. Additionally, the mark 114 may provide information to the physician regarding the lead connector 140, such as the type of lead connector 104, the location of a feature of the lead connector 140 or other information regarding the lead connector 140.

In accordance with one embodiment, the lead connector 140 comprises a radiopaque mark 142 that includes radiopaque material that is either applied to a surface of the connector 140, or formed integral to the connector 140. Suitable radiopaque materials, such as barium sulfate, may be used to form the radiopaque mark 142. In one embodiment, the radiological image of the mark 142 is distinguishable from other portions of the lead connector 140 that may be visible in the radiological image. The distinguishing features of the mark 142 may include a distinguishing shape, the brightness of the radiological image, or other distinguishing feature. In one embodiment, the mark 142 identifies the location of a feature of the lead connector 140, such as an opening or other feature.

One embodiment of the lead connector 140 comprises a tube 144, top and front schematic illustrations of which are respectively provided in FIGS. 7 and 8. In one embodiment, the tube 144 is formed of a flexible biocompatible material, such as silicone, which receives the distal end 132 of the lead 122 through an opening 146 or 148, as shown in FIG. 7. In one embodiment, the radiopaque mark 114 on the mesh 104 is positioned such that it indicates the location of the opening 146 and/or 148. In one embodiment, the inner diameter of the tube 144 is selected to provide an interference fit with the distal end 132 of the lead 122 to facilitate anchoring the lead 122 to the mesh 104. In one embodiment, the distal end 132 of the lead 122 may comprise external features that grip the tube 144 and provide the desired attachment to the tube 144, or assist in the attachment to the tube 144.

In accordance with some embodiments, the tube 144 includes one or more radiopaque marks 142. In one embodiment, the tube 144 includes a radiopaque mark 142 that indicates the location of the opening 146 and/or 148.

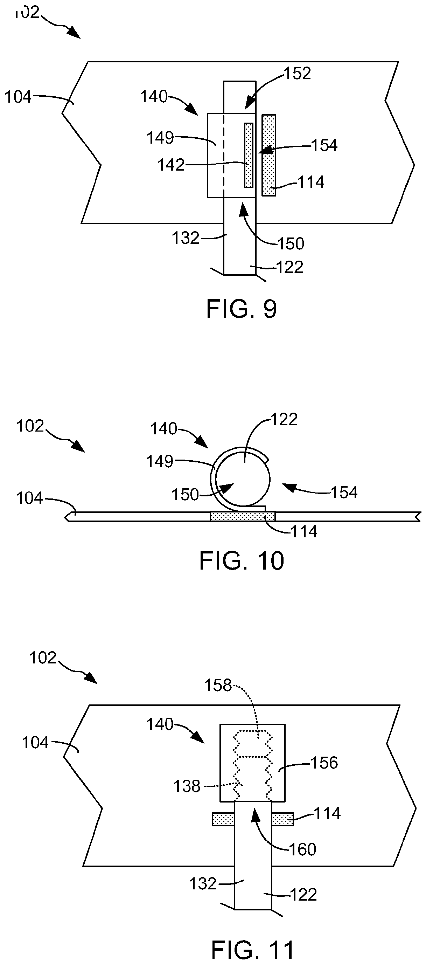

Another embodiment of the lead connector 140 is in the form of a clip 149 that receives or attaches to the distal end 132 of the electrode lead 122 to attach the electrode lead 122 to the mesh 104. Exemplary embodiments of the clip form of the lead connector 140 are illustrated schematically in the top and front views of FIGS. 9 and 10, respectively. In one embodiment, the clip lead connector 140 comprises an open end 150 and 152 and an open side 154. In one embodiment, the clip lead connector 140 flexes to expand the openings 150, 152 and 154 to allow for the insertion of the distal end 132 of the lead 122. In one embodiment, the clip lead connector 140 applies a pinching force to the distal end 132 of the lead 122 to secure the lead 122 to the mesh 104. In accordance with other exemplary embodiments, the distal end 132 of the electrode lead 104 may include features that assist in securing the lead 122 to the clip lead connector 140.

In one embodiment, the mesh 104 includes a radiopaque mark 114 that indicates the location of one of the openings of the clip lead connector 140, such as the location of the side opening 154, as shown in FIG. 9. Additionally, the clip lead connector 140 may include a radiopaque mark 142 to indicate the lead connector 140, or a feature thereof (e.g., side opening 154) as discussed above.

In accordance with another embodiment, the lead connector 140 comprises a threaded member 156 illustrated schematically in the top view of FIG. 11. In one embodiment, the threaded member 156 is attached to the mesh 104 using any suitable conventional technique. In one embodiment, the threaded member 156 comprises a threaded bore 158 and an opening 160 to the threaded bore 158. In one embodiment, the lead 122 comprises an anchor 138 that cooperates with the threaded bore 158 to secure the lead 122 to the threaded member 156 and, thus, the mesh 104. In one embodiment, the anchor 138 comprises a threaded cylindrical section that is screwed within the threaded bore 158 to secure the lead 122 to the threaded member 156, as shown in FIG. 11. Alternatively, the anchor 138 may comprise a threaded bore or socket, while the threaded member 156 comprises a threaded cylinder that is configured to be received within the threaded bore of the anchor 138.

In one embodiment, the mesh 104 includes a radiopaque mark 114 that indicates the location of the opening 160 of the threaded member 156, as shown in FIG. 11. Additionally, the threaded member 156 may include a radiopaque mark to indicate the location of the threaded member 156, the opening 160, or other feature of the connector 140.

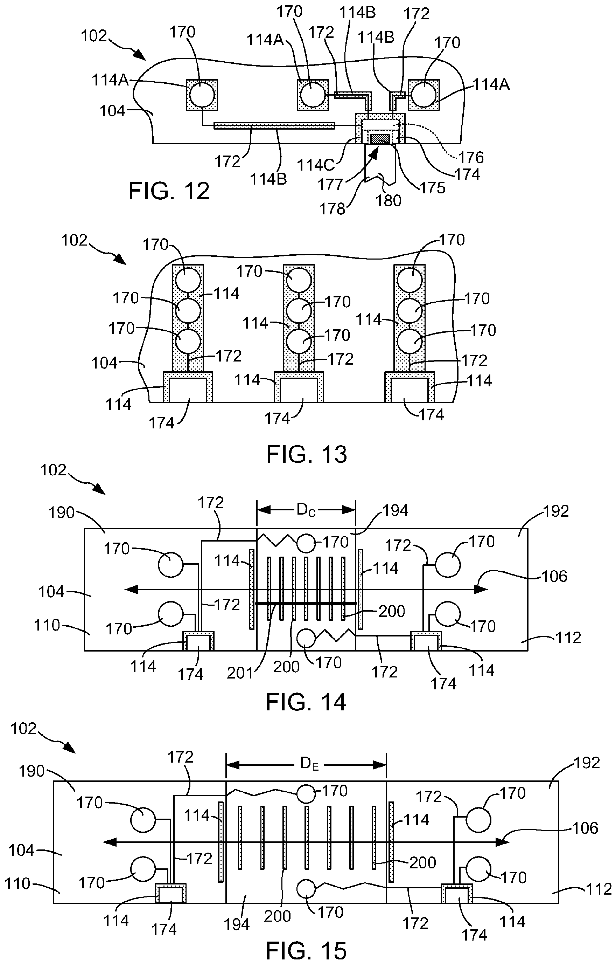

FIG. 12 is a schematically illustrated top view of a portion of the mechanical support 102 showing additional embodiments of the invention. In one embodiment, the mechanical support 102 comprises one or more electrodes 170, one or more electrical conductors 172, and an electrical connector 174 attached to the mesh 104. The electrical connector 174 is electrically coupled to the one or more electrodes 170 through the one or more conductors 172. In one embodiment, the electrodes 170 and the electrical conductors 172 are contained within a lead having a proximal end attached to the electrical connector 174.

In one embodiment, the mesh 104 includes one or more radiopaque marks 114 covering a subsection of the mesh 104 that indicates the location of the one or more electrodes 170, the one or more electrical conductors 172, and/or the electrical conductor 174. Thus, for example, the mechanical support 102 may include one or more radiopaque marks 114A that are located proximate to or surround the one or more electrodes 170 to indicate their location on the mesh 104. Likewise, the mechanical support 102 may include one or more radiopaque marks 114B that are located proximate to or around the one or more electrical conductors 172 to indicate their location on the mesh 104. Also, the mechanical support 102 may include one or more radiopaque marks 114C that are located around or proximate to the electrical connector 174 to indicate the location of the electrical connector, or a feature thereof, on the mesh 104. As with the lead connector 140, the electrical connector 174 may also include a radiopaque mark 175 (FIG. 12) indicating the location of the connector 174 or a feature of the connector 174, such as an opening 177 configured to receive an electrical lead, for example.

One embodiment of the electrical connector 174 comprises one or more electrical contacts 176, each of which is electrically coupled to one or more of the electrical conductors 172. The electrical connector 174 is configured to electrically couple to a distal end 178 of an electrical lead 180. The electrical lead 180 is generally formed in accordance with the lead 122, but may not include the electrodes or stimulation elements 130. In one embodiment, a body of the electrical lead encloses one or more electrical conductors, similar to the electrical conductors 136 within the body 128 of the electrode lead 122 (FIG. 3). In one embodiment, the electrical connector 174 comprises a socket that receives the distal end 178 of the electrical lead 180, as illustrated schematically in FIG. 12. Electrical contacts of the distal end 178 of the electrical lead 180 engage the electrical contacts 176 of the connector 174 when the distal end 178 is received within the socket of the connector 174. In one embodiment, the insertion of the distal end 178 of the electrical lead 180 into the socket of the electrical connector 174 triggers a mechanical coupling of the distal end 178 to the connector 174. This mechanical coupling can be achieved using any suitable conventional technique.

In one embodiment, the socket of the electrical connector 174 is protected from exposure to bodily fluids of the patient by a suitable septum or a membrane. When the electrical lead 180 is fed into the connector 174, it pierces the septum or membrane to achieve the desired electrical coupling. In one embodiment, the septum or membrane maintains a seal around the distal end 178 of the lead 180 to prevent body fluids from entering the socket of the connector 174.

FIG. 13 is a schematically illustrated top view of a portion of a mechanical support 102 in accordance with additional embodiments in the invention. As shown in FIG. 13, the mechanical support 102 may include a plurality of electrical connectors 174 attached to the mesh 104. In one embodiment, each of the electrical connectors 174 is electrically coupled to one or more electrodes 170 through one or more electrical conductors 172. Accordingly, each electrical connector 174 is electrically coupled to a different subset of the electrodes 170 that are attached to the mesh 104 of the mechanical support 102. In some embodiments, one or more radiopaque marks 114 are located around or proximate to each of the electrical connectors 174 to indicate their location on the mesh 104. In one embodiment, the mechanical support 102 comprises a plurality of radiopaque marks 114, each of which indicates a location of one of the electrical connectors 174 and the subset of electrodes 170 and / or electrical conductors 172 that are electrically coupled to the electrical connector 174. In accordance with this embodiment, a physician visualizing the radiological image of the mechanical support 102 can determine the subset of the electrodes 170 that are attached to each of the electrical connectors 174.

FIGS. 14-17 illustrate a mechanical support 102 in accordance with embodiments of the invention. In one embodiment, the section of mesh 104 comprises first and second portions 190 and 192 adjacent the first and second ends 110 and 112, respectively. The mesh portions 190 and 192 may comprise one or more embodiments described above with regard to the mesh 104 including the radiopaque marks 114, one or more lead connectors 140, one or more electrical connectors 174, and/or other features described herein.

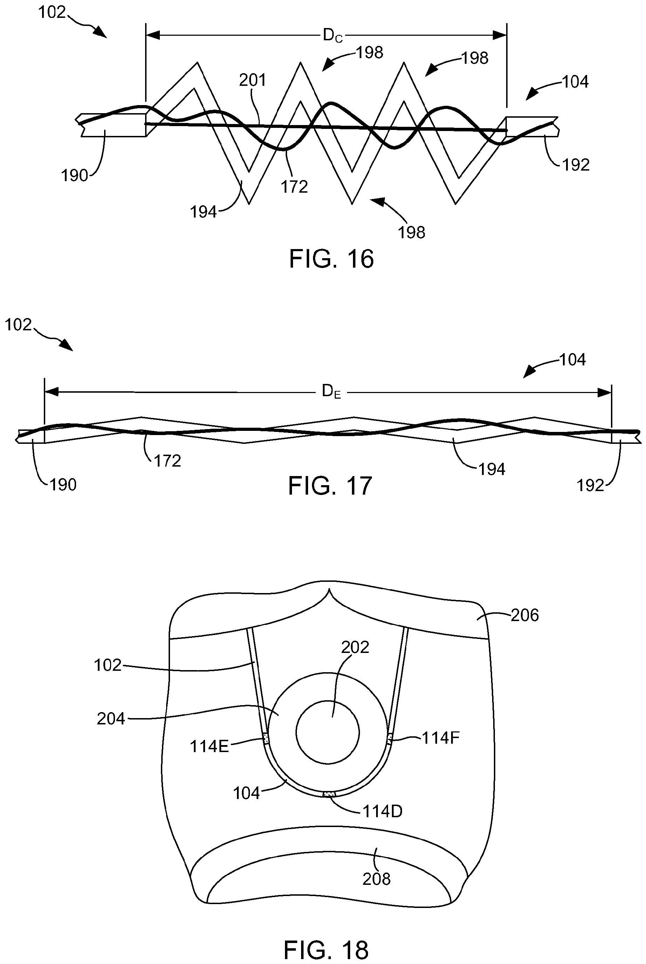

Additionally, the section of mesh 104 includes an expandable portion 194 that extends between the first and second portions 190 and 192. The expandable portion 194 comprises a compact state (FIGS. 14 and 16), in which the expandable portion 194 has a length D_{C} measured along the longitudinal axis 106, and an expanded state (FIGS. 15 and 17), in which the expandable portion 194 has a length D_{E} measured along the longitudinal axis 106 that is greater than the compact length D_{C}. In one embodiment, the expandable portion 194 is biased to maintain the compact state. That is, the quiescent state of the expandable portion 194 is the compact state. In one embodiment, the expandable portion 194 expands from the compact length D_{C} responsive to placing the support 102 in tension along the longitudinal axis 106. In one embodiment, this expansion of the expandable portion 194 along the longitudinal axis 106 does not occur responsive to plastic deformation of the material forming the expandable portion 194.

In one embodiment, the material forming the expandable portion 194 comprises the mesh used to form the first and second mesh portions 190 and 192. In accordance with other embodiments, the expandable portion 194 is formed of a different material than that used to form the mesh portions 190 and 192. In one embodiment, the expandable portion 194 comprises a plurality of folds 198 that facilitate the compact state of the expandable portion 194, as illustrated in FIG. 16. The application of the tensile force to the expandable portion 194 along the longitudinal axis 106 stretches out the folds 198 and expands the portion 194 to the expanded state, as shown in FIG. 17.

In one embodiment, the expandable portion 194 includes one or more electrodes 170 and corresponding electrical conductors 172, as shown in FIGS. 14 and 15. In one embodiment, the electrical conductors 172 in the expandable portion 194 are allowed to coil or fold when the expandable portion 194 is in the compact state, as illustrated in FIG. 16. As the expandable portion 194 is moved to the expanded state, the folds or coils of the electrical conductors 172 and the expandable portion 194 straighten out to allow for the expansion of the portion 194. In one embodiment, the electrical conductors 172 are interwoven within the material forming the expandable portion 194, as illustrated in FIGS. 16 and 17.

In one embodiment, the expandable portion 194 includes one or more radiopaque marks 200. The radiopaque marks 200 are formed in accordance with the embodiments described above with regard to the radiopaque marks 114. In one embodiment, the radiopaque marks 200 are equally spaced relative to each other along the longitudinal axis 106, as shown in FIG. 14. The expansion of the portion 194 from the compact to the expanded state causes the distance separating the marks 200 to increase, as shown in FIG. 15. This expansion of the marks 200 can be viewed in a radiological image and used to determine the amount expansion of the portion 194.

Alternatively, radiopaque marks 114 are provided on the first mesh section 190 and the second mesh section 192 adjacent the expandable portion 194, as shown in FIG. 14. The length of the expandable portion 194 along the axis 106 can be determined by measuring the distance separating the marks 114 on the first and second mesh sections 190 and 192.

In one embodiment, an absorbable suture 201 connects ends of the first and second mesh portions 190 and 192 together across the expandable portion 194. The absorbable suture 201 maintains the expandable portion 194 in the compact state. When the absorbable suture 201 dissolves, the expandable portion 194 becomes "unlocked" and is allowed to expand within the incorporated tissue. Thus, upon initial implantation of the support 102, the expandable portion 194 is maintained in the compact state by the suture 201. Over time the suture dissolves within the patient and the expandable portion 194 is released to expand freely within the incorporated tissue.

Additional embodiments are directed to methods of implanting the mechanical support 102 in the pelvic region of a patient for the purpose of treating a pelvic condition of the patient, such as urinary incontinence, fecal incontinence, sexual dysfunction, or other pelvic condition. In the first step of the method, an implantable mechanical support 102 formed in accordance with one or more of the embodiments described above, is provided. In one embodiment, the mechanical support comprises a section of mesh 104 having a longitudinal axis 106 and a radiopaque mark 114 covering a subsection of the mesh, such as illustrated in FIGS. 1 and 2.

Next, the mechanical support 102 is implanted adjacent a target site of the patient. In one embodiment, this implantation procedure is carried out in accordance with conventional techniques, such as those described above.

In order to simplify the discussion of the present invention, embodiments of the method will be described using the urethral sphincter of a female patient as the target site. Those skilled in the art understand that embodiments of the method may apply equally to other target sites within the pelvic region of male or female patients without departing from the spirit or scope of the invention.

FIGS. 18-22 are simplified views of a pelvic region of a female patient depicting the urethra 202, the urethra sphincter 204, the pubic bone 206, and the vaginal wall 208. In one embodiment of the method, the one or more radiopaque marks 114 are used to align the mechanical support 102, the mesh 104 and/or one or more features (e.g., electrodes 170, lead connectors 140, electrical connectors 174, etc.) to the target site or position these elements relative to the target site. In one embodiment, this is accomplished using a radiological technique to view the radiopaque marks 114 for final positioning of the mesh 104 relative to the target site. For instance, a physician may view a radiological image of the one or more radiopaque marks 114 on the mesh 104 and move the mesh 104 relative to the target site until the one or more marks 114 are aligned with the target site as desired. Accordingly, when the target site is a location adjacent the urethral sphincter 204, a central mark 114D, which indicates the center point of the mechanical support 102 or the mesh 104, may be aligned with the target site as desired with the aid of the radiological image, such as immediately below the target site, as illustrated in FIG. 18. Similarly, radiopaque marks 114E and 114F may indicate proper alignment of the mechanical support 102 or mesh 104 with the target site, such as by placing them on opposing sides of the target site, as shown in FIG. 18.

In one embodiment, the radiopaque marks 114 are formed of a color that is visibly distinguishable from the mesh 104. This allows the physician to align the marks 114 as desired relative to the target site, such as through the incision, without relying on a radiological image of the site.

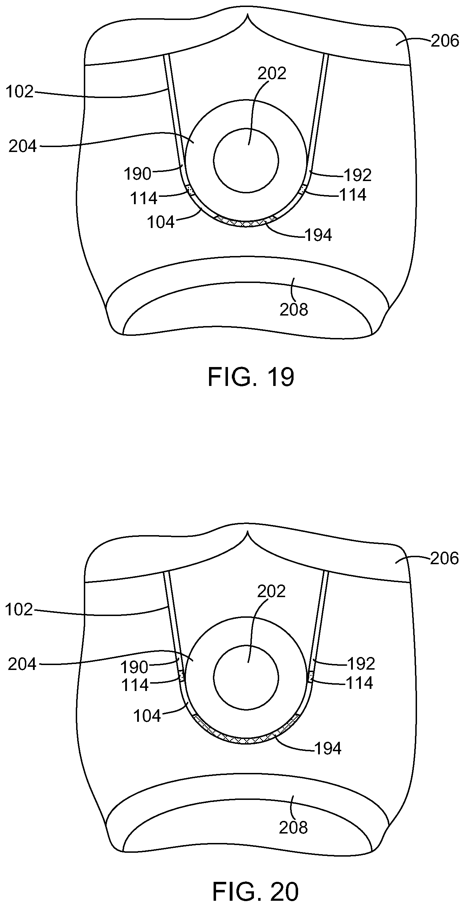

FIGS. 19 and 20 illustrate one embodiment of the method, in which the implantable support 102 comprises the expandable portion 194. In one embodiment, the physician positions the mesh 104 relative to the target site based on the one or more radiopaque marks 200 in the expandable portion 194 and/or the radiopaque marks 114 on the first or second mesh portions 190 and 192 using a radiological image or visually. In one embodiment, the physician places the first mesh section 190, the second mesh section 192 and the expandable section 194 in tension along the longitudinal axis 106. This expands the length of the expandable section 194, as indicated in FIG. 20. In one embodiment, the expandable section 194 is expanded until the one or more radiopaque marks 114 on the first mesh section 190 or the section mesh section 192 are positioned in a desired location relative to the target site. Once the one or more radiopaque marks 114 are positioned in the desired location relative to the target site, the ends 110 and 112 may be secured to the patient in accordance with conventional techniques, such as using anchors 108 (FIGS. 1 and 2) to fix the position of the mesh 104 relative to the target site.

Following the implantation of the mechanical support 102 formed in accordance with one or more of the embodiments described above, the patient is allowed to heal from the procedure for a week or more. The patient is also monitored to determined the effectiveness of the mechanical support 102 in treating the pelvic condition of the patient. Occasionally, the pelvic condition of the patient is not treated effectively, or new pelvic conditions arise that are desired to be treated using electrical stimulation. For instance, when the mechanical support 102 is initially implanted to treat stress incontinence, the patient may later develop urge incontinence, which may be treated using a stimulation therapy as discussed above.

Embodiments of the mechanical support 102 allow for the attachment of an electrode lead 122 or an electrical lead 180 depending upon the features present in the implanted mechanical support 102. For instance, when the mesh 104 of the mechanical support 102 does not comprise electrodes 170, an electrode lead 122 may be attached to the mesh 104 adjacent the desired target site to facilitate the delivery of electrical stimulation signals to the target site to treat the pelvic condition, as shown in FIG. 6. If the mechanical support 102 comprises one or more electrodes 170 on the mesh 104 and one or more electrical connectors 174, it is possible to deliver electrical stimulation signals to the target site through the one or more electrodes 170 by connecting one or more electrical leads 180 to one or more of the electrical connectors 174, as shown in FIG. 13. In one embodiment, these methods of facilitating electrical stimulation treatments of a pelvic condition are conducted a week or more following the implantation of the mechanical support 102.

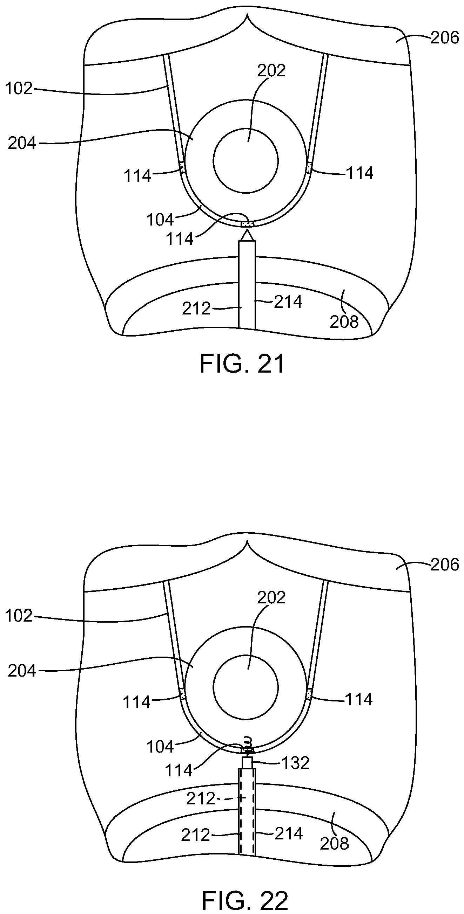

In one embodiment, an incision is made in the patient and a path is formed to the implanted mesh 104. For instance, an incision may be made in the vaginal wall 208 and a path may be formed through the tissue of the patient using a suitable tool, such as an introducer 212, as illustrated in FIG. 21. In one embodiment, the physician initially identifies a radiopaque mark 114 on the mesh 104 in a radiological image as a target mark for the path. In one embodiment, this target mark 114 corresponds to a section of the mesh 104, to which it is desired to attach an electrode lead 122. In accordance with another embodiment, the target mark 114 corresponds to the location of a lead connector 140. In accordance with yet another embodiment, the target mark 114 corresponds to the location of an electrical connector 174.

When the target mark 114 indicates a portion of the mesh 104, to which it is desired to attach the distal end 132 of an electrode lead 122, the distal end 132 is fed through a sheath 214 of the introducer 212 to the target mark 114, as shown in FIG. 22. In one embodiment, the electrode lead 122 comprises an anchor 138, such as a helical coil, that facilitates attachment of the lead 122 to the mesh 104. Following the attachment of the lead 122 to the mesh 104, the sheath 214 is removed from the patient leaving the lead 122 in place.

Following the attachment of the electrode lead 122 to the mesh 104, stimulation therapy may begin in accordance with conventional techniques. This may involve a preliminary testing period using an external stimulating device. If the preliminary testing period is successful, the lead 122 may be attached to an implantable stimulator device, such as that described above with reference to FIG. 3, in accordance with conventional methods.

When the implantable mechanical support 102 comprises one or more electrodes 170 and an electrical connector 174 on the mesh 104, an electrical lead 180 may be used to facilitate an electrical stimulation therapy for the patient. The process of attaching the electrical lead 180 to the electrical connector 174 is similar to that described above for the attachment of the electrode lead 122 to the mechanical support 102. That is, an incision is made in the patient, such as in the vaginal wall 208. Next, the distal end 178 of the electrical lead 180 is fed through the incision toward the implanted mechanical support 102. This may be accomplished using an introducer 212, as described above to form a path to the radiopaque mark on the mesh 104 corresponding to the electrical connector 174 or a radiopaque mark within the connector itself to which is it desired to attach the electrical lead 180. The physician locates the target radiopaque mark 114 corresponding to the desired electrical connector 174 by viewing a radiological image. Finally, the distal end of the electrical lead 180 is attached to the electrical connector 174, in accordance with the embodiments described above. The connection of the electrical lead 180 to the electrical connector 174 allows for electrical stimulation signals to be delivered to the patient through the electrical lead 180, the electrical connector 174, the conductors 172 and the electrodes 170. Preliminary testing of the electrical stimulation therapy can then commence followed by the implantation of the electrical lead 180 and a suitable stimulator device.

In one embodiment, the mechanical support 102 comprises a plurality of radiopaque marks 114, each of which identify one of a plurality of electrical connectors 174 on the mesh, as described above with reference to FIG. 13. In one embodiment, each of the radiopaque marks 114 also identifies a subset of the plurality of electrodes 170 or conductors 172 that correspond to the electrical connector 174. In one embodiment, the mechanical support 102 is positioned within the patient such that the subsets of the electrodes 170 are positioned to facilitate different types of stimulation therapies for the treatment of different pelvic conditions of the patient. For instance, one of the subsets of electrodes 170 may be positioned to provide treatment of urinary incontinence, while another subset of the electrodes 170 is positioned to provide stimulation therapy for the treatment of fecal incontinence or sexual dysfunction, for example.

In one embodiment, the physician identifies the electrical connector 174 using one of the radiopaque marks 114 that corresponds to the desired electrical stimulation treatment. The electrical lead 180 is then connected to the identified electrical connector 174 and stimulation therapy treatments may commence by delivering electrical stimulation signals to the one or more electrodes 170 through the electrical lead 180. As a result, the physician has the option of selecting among a plurality of stimulation therapies to apply to the patient subsequent to the implantation of the mechanical support 102 by simply attaching the electrical lead 180 to the electrical connector 174 corresponding to the desired treatment.

Although the present invention has been described with reference to preferred embodiments, workers skilled in the art will recognize that changes may be made in form and detail without departing from the scope of the invention.

## Claims

1. A mechanical support (102) of a pelvic treatment apparatus that is configured for implantation in the pelvic region of a patient, the mechanical support comprising:
a section of mesh (104) having a longitudinal axis (106);
at least one radiopaque (114) mark covering a subsection of the section of mesh;
one or more electrodes (170) attached to the section of mesh;
one or more electrical conductors (172) attached to the one or more electrodes and the mesh; and
an electrical connector (174) attached to the mesh and electrically coupled to the one or more electrodes through the one or more conductors, the electrical connector configured to electrically couple to a distal end of an electrical lead (180);
**characterized in that**:
the electrical connector overlays the section of mesh; and
the at least one radiopaque mark includes a radiopaque mark that underlays the electrical connector and indicates the location of the electrical connector on the section of mesh.

2. The mechanical support of claim 1, wherein the at least one radiopaque mark comprises a radiopaque line (116) on the section of mesh extending approximately perpendicularly to the longitudinal axis.

3. The mechanical support of claim 1, wherein the at least one radiopaque mark includes three or more radiopaque marks displaced from each other along the longitudinal axis.

4. The mechanical support of claim 1, further comprising a lead connector (140) attached to the mesh adjacent a lead connector radiopaque mark, the lead connector configured to couple an electrode lead (122) to the section of mesh.

5. The mechanical support of claim 4, wherein:
the lead connector comprises an opening (146, 148, 150, 152, 154) that is configured to receive the electrode lead; and
the lead connector radiopaque mark indicates the location of the opening.

6. The mechanical support of claim 5, wherein the lead connector is selected from the group consisting of a clip (149), a tube (144), and a threaded member (156).

7. The mechanical support of claim 1, wherein:
the one or more electrodes comprises a plurality of electrodes; and
the mechanical support comprises a plurality of electrical connectors (172) each electrically coupled to a different subset of the plurality of electrodes;
wherein the at least one radiopaque mark comprises a plurality of radiopaque marks each indicating a location of one of the electrical connectors.

8. The mechanical support of claim 7, wherein each of the plurality of radiopaque marks indicates a location of at least one of the subset of electrodes electrically coupled to the electrical connector and the electrical conductors electrically coupled to the electrical connector.

9. The mechanical support of claim 1, wherein the section of mesh comprises:
first and second portions (190, 192) respectively adjacent first and second ends (110, 112); and
an expandable portion (194) extending between the first and second portions, the expandable portion comprising a compact state, in which the expandable portion comprises a plurality of folds (198), and an expanded state, in which a length (D_{E}) of the expandable portion along the longitudinal axis (106) is greater than the length (D_{C}) of the expandable portion when in the compact state.

10. The mechanical support of claim 9, wherein the expandable portion does not undergo plastic deformation when in the expanded state.

11. The implantable mechanical support in accordance with any of claims 1-10, further comprising a sling including the section of mesh.

## Patentansprüche

1. Mechanische Stütze (102) einer Beckenbehandlungsvorrichtung, die konfiguriert ist, in der Beckengegend eines Patienten implantiert zu werden, wobei die mechanische Stütze aufweist:
einen Netzabschnitt (104) mit einer Längsachse (106);
mindestens eine strahlungsopake Markierung (114), die einen Teilabschnitt des Netzabschnitts bedeckt;
eine oder mehrere an dem Netzabschnitt angebrachte Elektroden (170);
einen oder mehrere an der einen oder den mehreren Elektroden und dem Netz angebrachte elektrische Leiter (172); und
einen elektrischen Verbinder (174), der an dem Netz angebracht ist und über den einen oder die mehreren Leiter elektrisch mit der einen oder den mehreren Elektroden gekoppelt ist, wobei der elektrische Verbinder konfiguriert ist, elektrisch mit einem distalen Ende einer elektrischen Leitung (180) zu koppeln;
**gekennzeichnet dadurch, dass**
der elektrische Leiter über dem Netzabschnitt liegt; und
die mindestens eine strahlungsopake Markierung eine strahlungsopake Markierung aufweist, die unterhalb des elektrischen Leiters angeordnet ist und den Ort des elektrischen Leiters an dem Netzabschnitt anzeigt.

2. Mechanische Stütze nach Anspruch 1, wobei die mindestens eine strahlungsopake Markierung eine strahlungsopake Linie (116) an dem Netzabschnitt aufweist, die sich ungefähr senkrecht zur Längsachse erstreckt.

3. Mechanische Stütze nach Anspruch 1, wobei die mindestens eine strahlungsopake Markierung drei oder mehr strahlungsopake Markierungen aufweist, die entlang der Längsachse in einem Abstand voneinander angeordnet sind.

4. Mechanische Stütze nach Anspruch 1, ferner aufweisend einen Leitungsverbinder (140), der angrenzend an eine strahlungsopake Leitungsverbinder-Markierung an dem Netz angebracht ist, wobei der Leitungsverbinder konfiguriert ist, eine Elektrodenleitung (122) mit dem Netzabschnitt zu koppeln.

5. Mechanische Stütze nach Anspruch 4, wobei
der Leitungsverbinder eine Öffnung (146, 148, 150, 152, 154) aufweist, die konfiguriert ist, die Elektrodenleitung aufzunehmen; und
die strahlungsopake Leitungsverbinder-Markierung den Ort der Öffnung anzeigt.

6. Mechanische Stütze nach Anspruch 5, wobei der Leitungsverbinder aus der folgenden Gruppe ausgewählt ist: eine Klammer (149), eine Röhre (144) und ein Gewindeelement (156).

7. Mechanische Stütze nach Anspruch 1, wobei
die eine oder mehreren Elektroden eine Vielzahl von Elektroden aufweist/aufweisen; und
die mechanische Stütze eine Vielzahl von elektrischen Verbindern (172) aufweist, die jeweils mit einer unterschiedlichen Teilmenge der Vielzahl von Elektroden gekoppelt sind;
wobei die mindestens eine strahlungsopake Markierung eine Vielzahl von strahlungsopaken Markierungen aufweist, die jeweils einen Ort eines der elektrischen Verbinder anzeigen.

8. Mechanische Stütze nach Anspruch 7, wobei jede der Vielzahl von strahlungsopaken Markierungen einen Ort der mit dem elektrischen Verbinder elektrisch gekoppelten Teilmenge von Elektroden und/oder (einen Ort) der elektrischen Leiter, die mit dem elektrischen Verbinder elektrisch gekoppelt sind, anzeigt.

9. Mechanische Stütze nach Anspruch 1, wobei der Netzabschnitt aufweist:
erste und zweite Abschnitte (190, 192) jeweils angrenzend an erste und zweite Enden (110, 112); und
einen expandierbaren Abschnitt (194), der sich zwischen den ersten und zweiten Abschnitten erstreckt, wobei der expandierbare Abschnitt aufweist: einen kompakten Zustand, in welchem der expandierbare Abschnitt mehrere Falten (198) aufweist, und einen expandierten Zustand, in welchem eine Länge (D_{E}) des expandierbaren Abschnitts entlang der Längsachse (106) größer ist als die Länge (D_{C}) des expandierbaren Abschnitts, wenn er in seinem kompakten Zustand ist.

10. Mechanische Stütze nach Anspruch 9, wobei der expandierbare Abschnitt keine plastische Verformung erleidet, wenn er in seinem expandierten Zustand ist.

11. Implantierbare mechanische Stütze nach einem der Ansprüche 1 bis 10, ferner aufweisend eine Schlinge, die den Netzabschnitt aufweist.

## Revendications

1. Support mécanique (102) d'un appareil de traitement de bassin qui est configuré pour être implanté dans la région pelvienne d'un patient, le support mécanique comprenant :
une section de maille (104) ayant un axe longitudinal (106) ;
au moins une marque radio-opaque (114) recouvrant une sous-section de la section de maille ;
une ou plusieurs électrodes (170) fixées sur la section de maille ;
un ou plusieurs conducteurs électriques (172) fixés aux une ou plusieurs électrodes et à la maille ; et
un connecteur électrique (174) fixé à la maille et électriquement couplé aux une ou plusieurs électrodes par le biais des un ou plusieurs conducteurs, le connecteur électrique étant configuré pour se coupler électriquement à une extrémité distale d'un fil électrique (180) ;
**caractérisé en ce que** :
le connecteur électrique recouvre la section de maille ; et
la au moins une marque radio-opaque comprend une marque radio-opaque qui est à la base du connecteur électrique et indique l'emplacement du connecteur électrique sur la section de maille.

2. Support mécanique selon la revendication 1, dans lequel la au moins une marque radio-opaque comprend une ligne radio-opaque (116) sur la section de maille s'étendant approximativement perpendiculairement à l'axe longitudinal.

3. Support mécanique selon la revendication 1, dans lequel la au moins une marque radio-opaque comprend trois marques radio-opaques ou plus déplacées les unes par rapport aux autres le long de l'axe longitudinal.

4. Support mécanique selon la revendication 1, comprenant en outre un connecteur de conducteur (140) fixé sur la maille adjacente à la marque radio-opaque de connecteur de conducteur, le connecteur de conducteur étant configuré pour coupler un conducteur d'électrode (122) à la section de maille.

5. Support mécanique selon la revendication 4, dans lequel :
le connecteur de conducteur comprend une ouverture (146, 148, 150, 152, 154) qui est configurée pour recevoir le conducteur d'électrode ; et
la marque radio-opaque de connecteur de conducteur indique l'emplacement de l'ouverture.

6. Support mécanique selon la revendication 5, dans lequel le connecteur de conducteur est sélectionné dans le groupe comprenant une attache (149), un tube (144) et un élément fileté (156).

7. Support mécanique selon la revendication 1, dans lequel :
les une ou plusieurs électrodes comprennent une pluralité d'électrodes ; et
le support mécanique comprend une pluralité de connecteurs électriques (172) chacun électriquement couplés à un sous-ensemble différent de la pluralité d'électrodes ;
dans lequel la au moins une marque radio-opaque comprend une pluralité de marques radio-opaques indiquant chacune un emplacement de l'un des connecteurs électriques.

8. Support mécanique selon la revendication 7, dans lequel chacune de la pluralité de marques radio-opaques indique un emplacement d'au moins l'une du sous-ensemble d'électrodes électriquement couplées au connecteur électrique et de conducteurs électriques électriquement couplés au connecteur électrique.

9. Support mécanique selon la revendication 1, dans lequel la section de maille comprend :
des première et seconde parties (190, 192) respectivement adjacentes aux première et seconde extrémités (110, 112) ; et
une partie expansible (194) s'étendant entre les première et seconde parties, la partie expansible comprenant un état compact dans lequel la partie expansible comprend une pluralité de plis (198), et un état expansé dans lequel une longueur (D_{E}) de la partie expansible le long de l'axe longitudinal (106) est supérieure à la longueur (D_{C}) de la partie expansible lorsqu'elle est à l'état compact.

10. Support mécanique selon la revendication 9, dans lequel la partie expansible ne subit pas de déformation plastique lorsqu'elle est à l'état expansé.

11. Support mécanique implantable selon l'une quelconque des revendications 1 à 10, comprenant en outre une sangle comprenant la section de maille.
